# FASCICULE DE BREVET EUROPEEN

(11) **EP 0 558 423 B1**
(45) Date de publication et mention de la délivrance du brevet: **22.05.1996**
(21) Numéro de dépôt: 93400509.1
(22) Date de dépôt: 26.02.1993
(51) Int. Cl.: A61K 7/48, A61K 7/00, A61K 7/043

(54) **Composition cosmétique ou dermatologique sous la forme d'une dispersion d'un composé organofluoré hydrocarboné dans une solution aqueuse d'un polymère filmogène hydrosoluble, susceptible de former des films composites**
Kosmetische oder dermatologische Zusammensetzung in Form einer Dispersion einer Organofluorkohlenwasserstoffverbindung in einer wässrigen Lösung eines wasserlöslichen filmbildenden Polymers, die Verbundfilme bilden kann
Cosmetic or dermatologic composition in the form of a dispersion of an organofluorinated hydrocarbon in an aqueous solution of a hydrosoluble film-forming polymer, capable of forming composite films

(30) Priorité: 27.02.1992 FR 9202295
(43) Date de publication de la demande: 01.09.1993
(73) Titulaire: L'OREAL, F-75008 Paris (FR)
(72) Inventeur: Arnaud, Pascal, F-75009 Paris (FR); Mellul, Myriam, F-94240 l'Hay les Roses (FR)
(74) Mandataire: Stalla-Bourdillon, Bernard

(56) Documents cités:
- EP-A- 0 158 996
- EP-A- 0 196 904
- EP-A- 0 360 292
- EP-A- 0 390 206
- WO-A-91/12793
- FR-A- 2 311 564
- GB-A- 2 190 393
- US-A- 4 059 688
- US-A- 4 087 394
- PATENT ABSTRACTS OF JAPAN vol. 016, no. 552 (C-1006)20 Novembre 1992
- PATENT ABSTRACTS OF JAPAN vol. 012, no. 349 (C-529)20 Septembre 1988

## Description

La présente invention a pour objet une composition cosmétique ou dermatologique sous forme d'une dispersion huile-dans-l'eau susceptible de former des films composites sur certains substrats, notamment sur les matières kératiniques, telles que la peau, les cheveux, les cils, les sourcils et les ongles, agissant en tant qu'écran à l'égard de solvants tels que l'eau.

L'invention concerne plus particulièrement une composition cosmétique ou dermatologique sous forme d'une dispersion d'une classe précise d'huiles fluorées à savoir de composés organofluorés hydrocarbonés dont 70 à 100 % des atomes d'hydrogène sont remplacés par des atomes de fluor, dans une solution aqueuse d'un polymère filmogène hydrosoluble.

La préparation de films composites nécessite l'association de polymères filmogènes à des substances censées modifier les caractéristiques du film obtenu, soit par association chimique soit par association physique (solubilisation ou dispersion).

Dans le cas d'une association chimique, le film composite est préparé par une réaction préalable de la substance à incorporer directement avec le polymère filmogène tel que décrit par exemple dans la demande de brevet français n° 76.15335 (n° de publication 2.311.564).

Dans le cas d'une association physique, on sait que l'on peut obtenir des films composites formés par séchage contenant certaines substances qui se retrouvent "solubilisées" dans le film au sens où elles sont solubilisées dans la phase aqueuse à partir de laquelle, par application sur un substrat, tel que les matières kératiniques, on obtient le film.

Parmi ces substances on citera les plastifiants, les principes actifs et les colorants hydrosolubles.

On connaît en outre des films composites contenant des substances non hydrosolubles qui y sont incorporées, également lors de la préparation du film, par dispersion dans la phase aqueuse avant que celle-ci ne soit appliquée sur un substrat tel que la peau. Parmi ces substances on citera les charges, les pigments ou des substances lipophiles telles que les huiles.

Or, à la connaissance de la Société déposante, l'incorporation d'huiles fluorées sous forme dispersée dans une solution aqueuse d'un polymère filmogène hydrosoluble en vue d'obtenir un film composite, n'a pas été décrite jusqu'à présent.

Il existe toutefois des dispersions d'huiles fluorées dans des solutions aqueuses de polymères hydrosolubles.

On citera à cet égard la demande de brevet européen n° 360.292 qui décrit des émulsions destinées à nettoyer la peau mais qui n'ont pas la propriété de former des films protecteurs.

La Société déposante a en effet pu mettre en évidence que l'élimination de l'eau des émulsions décrites dans cette demande de brevet, par évaporation à température ambiante, ne permet pas de conduire à la formation d'un film homogène.

Il est également connu d'après EP-A-0 196 904 et EP-A-O 390 206 d'utiliser dans des compositions cosmétiques à titre d'agent filmogène des perfluoropolyéthers dispersés dans une phase organique ou aqueuse.

Après diverses études, on a constaté que suite à un choix particulier d'une classe de composés fluorés, on pouvait obtenir d'une manière inattendue et surprenante, une composition sous forme d'une dispersion conduisant à des films composites dans lesquels les substances incorporées, à savoir les composés fluorés, restaient parfaitement dispersées à l'intérieur du film composite une fois obtenu.

En effet, on a découvert que par l'emploi de certains composés organofluorés hydrocarbonés, à savoir des hydrocarbures dont 70 à 100 % des atomes d'hydrogène ont été remplacés par des atomes de fluor, les compositions obtenues étaient très satisfaisantes en ce qui concerne la qualité et la stabilité des films obtenus.

La présente invention a donc pour objet une composition cosmétique ou dermatologique sous forme d'une dispersion huile-dans-l'eau susceptible de former un film composite par évaporation de la phase aqueuse continue caractérisée par le fait qu'elle comprend une phase fluorée constituée d'au moins un composé organofluoré hydrocarboné qui est un hydrocarbure dont au moins 70 % et de préférence au moins 95 % des atomes d'hydrogène sont substitués par des atomes de fluor, ladite phase étant dispersée dans une solution aqueuse d'au moins un polymère filmogène hydrosoluble exempt de motifs de formule suivante : 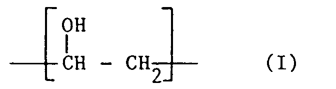
Grâce au choix des composés organofluorés hydrocarbonés tels que définis ci-dessus il est maintenant possible d'obtenir des films composites les contenant et ayant des caractéristiques intéressantes telles qu'une augmentation de la souplesse et de l'élasticité.

On a pu par ailleurs mettre en évidence, que les films obtenus à partir de la composition selon l'invention, présentaient une diminution de la sensibilité à l'eau, ce qui les rend moins poisseux à son contact en diminuant leur vitesse de redissolution.

En outre, on a remarqué que les films obtenus à partir de la composition selon l'invention, possèdaient de bonnes propriétés sensorielles tactiles, la présence des composés organofluorés hydrocarbonés conduisant à un toucher plus doux et plus agréable du film.

Les compositions selon l'invention se présentent notamment sous forme de masques de beauté, de gels amincissants, de crèmes protectrices, de gels coiffants, d'eye-liners, de mascaras éventuellement colorés, de vernis à ongles aqueux, de laques à l'eau et de produits de coloration temporaire des cheveux.

Les composés organofluorés hydrocarbonés utilisés selon l'invention sont de préférence à l'état liquide à température ambiante et sous pression normale. Toutefois, on peut également utiliser des composés organofluorés hydrocarbonés solides à température ambiante dans la mesure où ils peuvent être solubilisés dans un composé organofluoré hydrocarboné liquide à température ambiante.

Selon l'invention, la phase fluorée peut être présente en une proportion comprise entre 0,01 et 70 % et de préférence entre 0,1 et 50 % en poids par rapport au poids total de la composition.

Le composé organofluoré hydrocarboné utilisé selon l'invention peut être aliphatique ou aromatique, saturé ou insaturé, ayant un squelette carboné linéaire, ramifié ou cyclique, contenant éventuellement des hétéroatomes tels que l'azote et l'oxygène et lorsque le squelette carboné contient de l'oxygène, il s'agit de préférence d'un squelette cyclique.

Parmi les composés organofluorés hydrocarbonés utilisés selon l'invention, on peut citer (A) : les composés organo perfluorés hydrocarbonés, à savoir ceux ayant un taux de substitution des atomes d'hydrogène par des atomes de fluor de 100 % ainsi que (B) : les composés organiques partiellement fluorés hydrocarbonés, à savoir ceux ayant un taux de substitution des atomes d'hydrogène par des atomes de fluor inférieur à 100 % mais supérieur à 70 %.
(A). Parmi les composés organo perfluorés hydrocarbonés utilisés selon l'invention, on peut citer : (1) les perfluoroalcanes (2) les perfluorocycloalcanes, (3) les perfluoro(alkylcycloalcanes), (4) les perfluoropolycycloalcanes, (5) les hydrocarbures perfluorés aromatiques (les perfluoroarènes) et (6) les composés organo perfluorés hydrocarbonés comportant au moins un hétéroatome.
   (1). Parmi les perfluoroalcanes, on peut citer la série des alcanes linéaires tels que le perfluorooctane, le perfluorononane ou le perfluorodécane.
   (2). Parmi les perfluorocycloalcanes et (3) les perfluoro(alkylcycloalcanes), on peut citer la perfluorodécaline vendue sous la dénomination de "FLUTEX PP5" par la Société RHONE POULENC, la perfluoro(méthyldécaline), les perfluoro(C₃-C₅alkylcyclohexanes) tels que le perfluoro(butylcyclohexane).
   (4). Parmi les perfluoropolycycloalcanes on peut citer les dérivés de bicyclo [3.3.1] nonane tel que le perfluorotriméthylbicyclo [3.3.1] nonane, les dérivés de l'adamantane tels que le perfluorodiméthyladamantane et les dérivés perfluorés de phénanthrène hydrogéné tel que le tétracosafluoro-tétradécahydrophénanthrène.
   (5). Parmi les perfluoroarènes, on peut citer les dérivés perfluorés du naphtalène comme le perfluoronaphtalène et le perfluorométhyl-1-napthtalène.
   (6). Parmi les composés organo perfluorés hydrocarbonés comportant au moins un hétéroatome, on peut citer les perfluoro(tert-amines) comme la perfluoro-tributylamine, ou des composés hétérocycliques saturés, substitués par des restes alkyles, comme les perfluoro(alkyltétrahydropyrannes) tels que le perfluoro(hexyltétrahydropyranne), les perfluro(alkyltétrahydrofurannes) tels que le perfluoro(heptyltetrahydrofuranne) et le perfluoro(butyl- tétrahydrofuranne) ou les dérivés de la morpholine tels que la perfluoro(N-pentylmorpholine).
(B). Parmi les composés organiques partiellement (> 70 %) fluorés hydrocarbonés on peut citer ceux de formule suivante :

   (RF)ₓ-(A)_{y}-(RH)_{z} (II)

   dans laquelle :
   x peut être 1, 2 ou 3 ;
   y peut être 0 ou 1 ;
   z peut être 0, 1, 2 ou 3 ;
   y et z ne pouvant être simultanément zéro,
   et lorsque z = 0, alors y = 1 et x = 2 ou 3;
   RF représente un radical fluoré aromatique ou aliphatique, saturé ou insaturé, dont la chaine peut être linéaire, ramifiée ou cyclique, la channe pouvant être éventuellement fonctionnalisée et/ou pouvant être interrompue par des atomes divalents tels que l'oxygène ou le soufre, ou trivalents comme l'azote, la channe pouvant en outre être substituée par des atomes d'hydrogène ou d'autres halogènes, à condition que pas plus d'un de ces substituants, autres que le fluor, ne soit présent pour 2 atomes de carbone ;
   RH représente un radical hydrocarboné aliphatique ou aromatique, saturé ou insaturé, dont la channe peut être linéaire, ramifiée ou cyclique éventuellement fonctionnalisée et/ou interrompue par des atomes divalents tels que l'oxygène ou le soufre, ou par des atomes trivalents comme l'azote ; et A représente un radical di, tri ou quadrivalent tel que :
   〉C〈, 〉CH- , -N〈, -CO-N〈, -SO₂N〈, 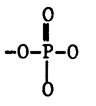 les structures cycliques, aliphatiques ou aromatiques ou les insaturations éthyléniques.

Par fonctionnalisé, on entend selon l'invention, une substitution du squelette, intercalaire, terminale ou pendante, par au moins un groupement organique fonctionnel comme une fonction alcool, thiol, acide, carbonyle, sulfoxyde, ester, amide, amine, phosphate, éthylénique, acéthylénique, et énamine ou sulfonamide.

Par insaturation éthylénique, on entend par exemple :
〉C = C〈, 〉C = CH- ou -CH=CH-
De préférence, RH représente un radical alkyle linéaire ou ramifié en C₁-C₂₂ ou un mélange de radicaux alkyles linéaires ou ramifiés en C₁-C₂₂, un radical aryle en C₆-C₁₀ ou un radical aralkyle en C₇-C₁₅.

De préférence, RF représente un radical perfluoroalkyle ayant de 4 à 22 atomes de carbone.

A titre d'exemple de composés organiques partiellement (> à 70 %) fluorés hydrocarbonés utilisables selon l'invention, on peut citer les produits ayant les formules suivantes :
(a) 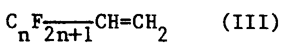 où n est 6 ou 8
(b) 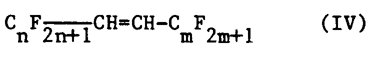 où n et m sont compris entre 2 et 10 et la somme n + m est égale ou supérieure à 8,
(c) 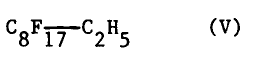 (d) 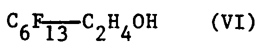 et
(e)

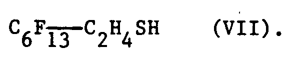

Le polymère filmogène hydrosoluble utilisé selon l'invention est présent de manière générale en une proportion comprise entre 0,05 et 55 % et de préférence entre 0,5 et 30 % en poids par rapport au poids total de la composition.

Parmi ceux-ci on citera :
- les dérivés de kératine, tels que les hydrolysats de kératine et les kératines sulfoniques. Parmi les kératines sulfoniques, on peut citer celle vendue sous la dénomination de "MEXORYL SU" par la Société CHIMEX ;
- les dérivés de chitine ou de chitosane anioniques, cationiques, amphotères ou non ioniques. Parmi les dérivés de chitosane, on peut citer le pyrrolidone carboxylate de chitosonium vendu sous la dénomination de de "KYTAMER PC" par la Société AMERCHOL ;
- les dérivés de cellulose, tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose. Parmi les dérivés de cellulose, on peut citer l'hydroxyéthylcellulose vendue sous la dénomination de "CELLOSIZE QP 4400 H" par la Société AMERCHOL,les dérivés d'éthers de cellulose comportant des groupements ammonium quaternaires tels que les polymères commerciaux vendus sous la dénomination "JR" par la Société UNION CARBIDE ou les dérivés de cellulose cationiques vendus sous les dénominations "CELQUAT L200" et "CELQUAT H100" par la Société NATONAL STARCH ;
- les polymères acryliques, tels que les polyacrylates et les polyméthacrylates, ainsi que les copolymères acryliques ;
- les copolymères vinyliques non hydroxylés, tels que la polyvinylpyrrolidone et ses copolymères, le copolymère de l'éther méthylvinylique et de l'anhydride maléique, éventuellement monoestérifié ou monoamidifié et le copolymère de l'acétate de vinyle et de l'acide crotonique. Parmi les copolymères vinyliques non hydroxylés, on peut citer la polyvinylpyrrolidone vendue sous la dénomination "LUVISKOL K90 PULVER" par la Société BASF et le copolymère polyvinylpyrrolidone/acétate de vinyle vendu sous la dénomination "PVP/VA S630" par la Société GAF ;
- les polymères naturels tels que les gommes arabiques, la gomme de guar et de caroube, les dérivés du xanthane et la gomme de karaya, les alginates et les carraghénates, les glycoaminoglycanes, l'acide hyaluronique et ses dérivés. Parmi les dérivés de xanthane, on peut citer celui vendu sous la dénomination de "RHODOPOL SC" par la Société RHONE POULENC ;
- les polyuréthanes et leurs copolymères.

Les polymères filmogènes hydrosolubles utilisés selon l'invention, constituent la matrice du film composite dans laquelle se retrouve à l'état de dispersion le ou les composé(s) organofluoré(s) hydrocarboné(s).

Selon un autre mode de réalisation, la phase fluorée comprend au moins un composé fluoré additionnel qui peut être un perfluoropolyéther et/ou un composé organofluoré hydrocarboné résultant d'un hydrocarbure dont moins de 70 % des atomes d'hydrogène ont été substitués par des atomes de fluor. Ces composés additionnels peuvent être fonctionnalisés ou non-fonctionnalisés et seront solubilisés dans le (ou les) composé(s) organofluoré(s) hydrocarboné(s) dont 70 à 100 % des atomes d'hydrogène sont remplacés par des atomes de fluor. Par l'emploi du terme "fonctionnalisé" on entend une substitution de la chaîne par divers groupements organiques.

Parmi les perfluoropolyéthers non fonctionnalisés on peut citer :
1) Ceux ayant la formule suivante : 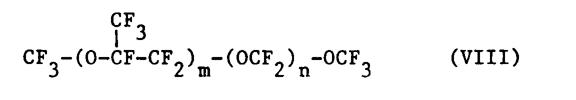 dans laquelle : m/n = 5 à 40, le poids moléculaire moyen étant supérieur à 500 et de préférence compris entre 1 000 et 10 000.
   Parmi ceux-ci, on peut citer ceux vendus par la Société MONTEFLUOS sous les dénominations de "GALDEN", de "FOMBLIN Y", et de "FOMBLIN HC", et notamment le "FOMBLIN HC25" pour lequel m/n = 20 à 40 et PM = 3.200).
2) Ceux ayant la formule suivante :

   CF₃-(O-CF₂-CF₂)ₚ-(OCF₂)_{q}-OCF₃ (IX)

   dans laquelle : p/q est de 0,5 à 1,5, le poids moléculaire moyen étant supérieur à 500 et de préférence compris entre 1 000 et 10 000
   Parmi ceux-ci on peut citer celui vendu sous la dénomination "FOMBLIN Z" par la Société MONTEFLUOS ;
3) Ceux ayant la formule suivante : 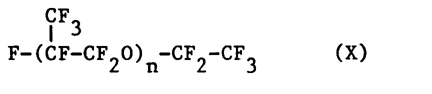 dans laquelle : n est un nombre entier de 4 à 500.
   Parmi ceux-ci on peut citer celui vendu sous la dénomination de "KRYTOX" par la Société DUPONT DE NEMOURS.
4) Ceux ayant la formule suivante : 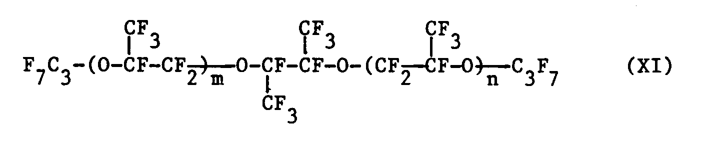 dans laquelle : n et m sont des nombres entiers de 0 à 3.

Parmi ceux-ci on peut citer celui vendu sous la dénomination de "HOSTINERT" par la Société HOECHST.

Parmi les perfluoropolyéthers fonctionnalisés on peut citer ceux ayant la formule suivante : 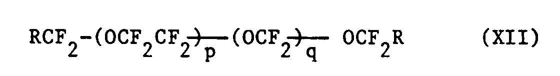
dans laquelle : p/q est de 0,5 à 1,5,
R représente un reste -COOCH₃, -CH₂OH,
-CH₂O-CH₂-CHOH-CH₂OH ou -CH₂-(OCH₂-CH₂)ₜ-OH où t est 1 ou 2,
le poids moléculaire moyen étant supérieur à 500 et de préférence compris entre 1 000 et 10 000.

Parmi ceux-ci on peut citer ceux vendus sous les dénominations de "FOMBLIN Z-DOL" (R = -CH₂OH) et de "FOMBLIN Z-DOL-TX" (R = -CH₂(OCH₂CH₂)ₜOH où t est 1 ou 2) par la Société MONTEFLUOS.

Parmi les composés organofluorés hydrocarbonés résultant d'un hydrocarbure dont moins de 70 % des atomes d'hydrogène ont été substitués par des atomes de fluor, on peut citer ceux de formule :

(RF)ₓ-(A)_{y}-(RH)_{z} (XIII)

dans laquelle : x, y, z, RF, A et RH ont les significations indiquées ci-dessus et par exemple les composés de formule : 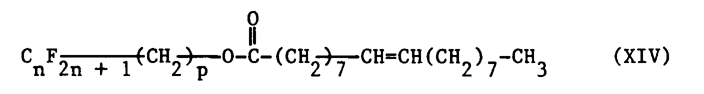
dans laquelle : n est un nombre entier égal à 6 ou 8 et p est 1 ou 2, vendus sous la dénomination "NOFABLE FO" par la Société NIPPON OIL & FATS CO.

Selon l'invention le composé fluoré additionnel peut être présent à raison de 0,01 à 50 % en poids et de préférence entre 0,1 à 20 % en poids par rapport au poids total de la phase fluorée.

Les compositions cosmétiques ou dermatologiques selon l'invention peuvent contenir dans la phase aqueuse au moins un additif communément utilisé en cosmétique ou en dermatologie, tel que par exemple un actif hydrosoluble tel qu'un agent hydratant, en particulier un composé polyhydroxylé.

On citera par exemple le glycérol, le sorbitol ou le D-panthénol qui sont également utiles pour plastifier les films obtenus par étalement des compositions cosmétiques ou dermatologiques selon l'invention. Parmi d'autres additifs, on peut citer des agents amincissants, des agents anti-rides, des stimulants, des revitalisants, des raffermissants et des adoucissants, de préférence dans des proportions comprises entre 0,01 et 30 % en poids par rapport au poids total de la composition.

Les compositions cosmétiques ou dermatologiques selon l'invention peuvent également contenir dans la phase aqueuse des épaississants tels que par exemple le "CARBOPOL" (vendu par la Société GOODRICH), des huiles émulsionnées, des polymères non filmogènes, des conservateurs, des séquestrants, des agents anti-mousse, des parfums, des colorants, des pigments, des modificateurs de pH ou encore des charges.

Les compositions selon l'invention, peuvent être obtenues de la manière suivante :

On prépare dans un premier temps une solution aqueuse dans laquelle le polymère filmogène hydrosoluble est solubilisé en une proportion comprise entre 0,05 et 55 % en poids. Si désiré, des additifs tels que ceux mentionnés ci-dessus ou bien d'autres peuvent être ajoutés à ce stade de la préparation.

A partir de la solution aqueuse ainsi préparée, on disperse alors le ou les composé(s) organofluoré(s) hydrocarboné(s), éventuellement en mélange avec au moins un composé fluoré additionnel, de manière classique en une proportion telle qu'indiquée ci-dessus à l'aide d'un agitateur de type RAYNERI ou MORITZ à une température proche de la température ambiante.

La taille des particules ainsi obtenues dans la composition selon l'invention peut varier entre 0,2 et 50 µm, de préférence entre 0,5 et 10 µm.

La composition filmogène selon l'invention peut être appliquée sur un support kératinique adapté tel que la peau, les cheveux, les cils, les sourcils ou les ongles sous forme d'une couche homogène de faible épaisseur puis on laisse évaporer l'eau de la phase continue à température ambiante afin d'obtenir un film composite. En l'absence de matière colorante et de charge, le film ainsi formé apparaît translucide à opaque suivant le pourcentage de de composé(s) organofluoré(s) hydrocarbonés qu'il renferme. Sa surface est parfaitement homogène à l'état sec, et ne colle pas. On observe après séchage du film qu'aucune démixtion de la phase fluorée ne s'est produite et que celle-ci reste dispersée dans le film sous la forme de gouttelettes qui de manière surprenante, ont un diamètre sensiblement égal à celui des particules de la dispersion aqueuse avant séchage, à savoir de 0,2 à 50 µm.

Le temps d'évaporation et de séchage du film obtenu à partir des compositions filmogènes selon l'invention, est de l'ordre de 1 à 15 minutes et de préférence de 2 à 10 minutes.

Selon un mode de réalisation préféré, la formation du film peut-être accélérée par addition, à la composition cosmétique, d'un alcool de bas poids moléculaire tel que l'éthanol ou l'isopropanol en une proportion comprise entre 0,01 et 20 %.

Les exemples qui suivent illustrent l'invention sans pour autant la limiter.

### EXEMPLES DE COMPOSITIONS FILMOGENES COSMETIQUES ET DERMATOLOGIQUES

Les exemples suivants décrivent des compositions dont le mode de préparation est classique.

Dans chacun de ces exemples, la phase fluorée est dispersée sous agitation dans la solution aqueuse contenant le ou les polymères filmogènes hydrosolubles et éventuellement des additifs, de préférence à une température proche de la température ambiante.

### EXEMPLE 1 : Gel coiffant

| | |
|---|---|
| - "CARBOPOL 940" | 0,25 g |
| - Triéthanolamine qs pH = 6,5 | |
| - Polymère hydrosoluble "PVP/VA S 630" | 1,00 g |
| - Composé organofluoré hydrocarboné "FLUTEC PP5" | 1,00 g |
| - Alcool éthylique | 10,00 g |
| - Conservateur qs | |
| - Eau qsp | 100,00 g |

Appliquée sur les cheveux, la composition forme un film qui permet un maintien naturel de la coiffure.

### EXEMPLE 2 : Eye-liner

| | |
|---|---|
| - "CARBOPOL 941" | 0,30 g |
| - Triéthanolamine qs pH = 6,5 | |
| - Polymère hydrosoluble "CELLOSIZE QP 4400 H" | 0,50 g |
| - Composé organofluoré hydrocarboné "FLUTEC PP5" | 3,00 g |
| - Composé organofluoré hydrocarboné"FOMBLIN HC25" | 0,30 g |
| - Glycérol | 3,60 g |
| - Oxyde de fer noir | 12,00 g |
| - Conservateur qs | |
| - Eau qsp | 100,00 g |

Appliquée sur la paupière, au ras des cils, la composition sèche rapidement et forme un film homogène qui permet de dessiner un trait régulier.

### EXEMPLE 3 : Vernis à ongles

| | |
|---|---|
| - Polymère hydrosoluble : dispersion aqueuse de polyuréthane | 38,0 g |
| - "CARBOPOL 940" | 0,08 g |
| - Pigments | 0,8 g |
| - Composé organofluoré hydrocarboné "FLUTEC PP5" | 5,0 g |
| - Ethanol | 5,0 g |
| - Conservateur qs | |
| - Eau qsp | 100,0 g |

Ce vernis s'applique facilement sur les ongles et sèche sans relargage de l'huile fluorée. Le film obtenu a un toucher doux et agréable.

## Revendications

1. Composition cosmétique ou dermatologique sous la forme d'une dispersion huile-dans-l'eau susceptible de former un film composite par évaporation de la phase aqueuse caractérisée par le fait qu'elle comprend une phase fluorée constituée d'au moins un composé organofluoré hydrocarboné qui est un hydrocarbure dont au moins 70 % et de préférence au moins 95 % des atomes d'hydrogène ont été substitués par des atomes de fluor, ladite phase étant dispersée dans une solution aqueuse d'au moins un polymère filmogène hydrosoluble exempt de motifs de formule suivante :

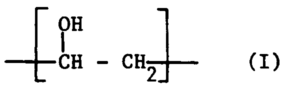

2. Composition selon la revendication 1, caractérisée par le fait que la phase fluorée est présente à une concentration comprise entre 0,01 et 70 % et de préférence entre 0,1 et 50 % en poids par rapport au poids total de la composition.

3. Composition selon la revendication 1 ou 2, caractérisée par le fait que la phase fluorée renferme au moins un composé organofluoré hydrocarboné solide à température ambiante solubilisé dans au moins un composé organofluoré hydrocarboné liquide à température ambiante.

4. Composition selon l'une quelconque des revendications précédentes caractérisée par le fait que le composé organofluoré hydrocarboné est un composé organo perfluoré hydrocarboné choisi parmi les perfluoroalcanes, les perfluorocycloalcanes, les perfluoro(alkylcycloalcanes), les perfluoropolycycloalcanes, les perfluoroarènes et les composés organo perfluorés hydrocarbonés comportant au moins un hétéroatome.

5. Composition selon la revendication 4, caractérisée par le fait que le composé organo perfluoré hydrocarboné est choisi parmi le perfluorooctane, le perfluorononane, le perfluorodécane, la perfluorodécaline, la perfluoro(méthyldécaline), le perfluoro(butylcyclohexane), le perfluorotriméthylbicyclo [3.3.1] nonane, le perfluorodiméthyladamantane, le tétracosafluorotétradécahydrophénanthrène, le perfluoronapthalène et le perfluorométhyl-1-naphtalène.

6. Composition selon la revendication 4, caractérisée par le fait que le composé organo perfluoré hydrocarboné comportant au moins un hétéroatome est choisi parmi les perfluoro(tert-amines), les perfluoro (alkyltétrahydrofurannes), les perfluoro(alkyltétrahydropyrannes) et les dérivés perfluorés de la morpholine.

7. Composition selon la revendication 6, caractérisée par le fait que le composé organo perfluoré hydrocarboné comportant au moins un hétéroatome est choisi parmi la perfluorotributylamine, le perfluoro(butyltétrahydrofuranne), le perfluoro(hexyltétrahydropyranne) et la perfluoro-(N-pentylmorpholine).

8. Composition selon l'une quelconque des revendications 1 à 3, caractérisée par le fait que le composé organofluoré hydrocarboné est un composé organique partiellement fluoré hydrocarboné de formule :
(RF)ₓ-(A)_{y}-(RH)_{z} (II)
dans laquelle :
x peut être 1,2 ou 3 ;
y peut être 0 ou 1 ;
z peut être 0, 1, 2 ou 3 ;
y et z ne pouvant être simultanément zéro,
et lorsque z = 0, alors y = 1 et x = 2 ou 3 ;
RF représente un radical fluoré aromatique ou aliphatique, saturé ou insaturé, dont la chaîne peut être linéaire, ramifiée ou cyclique, la chaîne pouvant être éventuellement fonctionnalisée et/ou pouvant être interrompue par des atomes divalents tels que l'oxygène ou le soufre, ou trivalents comme l'azote la channe pouvant en outre être substituée par des atomes d'hydrogène ou d'autres halogènes, à condition que pas plus d'un de ces substituants, autres que le fluor, ne soit présent pour 2 atomes de carbone,
RH représente un radical hydrocarboné aliphatique ou aromatique, saturé ou insaturé, dont la channe peut être linéaire, ramifiée ou cyclique éventuellement fonctionnalisée et/ou interrompue par des atomes divalents tels que l'oxygène ou le soufre, ou par des atomes trivalents comme l'azote,
et A représente une liaison covalente quand y = 0 et quand y est différent de 0, A représente un radical di- ou polyvalent.

9. Composition selon la revendication 8, caractérisée par le fait que le composé hydrocarboné partiellement fluoré hydrocarboné répond à l'une des formules suivantes :
a) 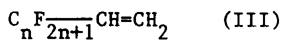 dans laquelle : n est 6 ou 8
b) 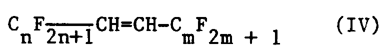 dans laquelle : n et m sont indépendament des nombres entiers entre 2 et 10 et la somme n + m est supérieure ou égale à 8
c)
C₈F₁₇-C₂H₅ (V),
d) 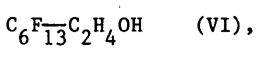 et
e)

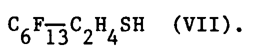

10. Composition selon l'une quelconque des revendications précédentes, caractérisée par le fait que le polymère filmogène hydrosoluble est présent en une proportion comprise entre 0,05 et 55 % et de préférence entre 0,5 et 30 % en poids par rapport au poids total de la composition.

11. Composition selon l'une quelconque des revendications précédentes, caractérisée par le fait que le polymère filmogène hydrosoluble est choisi parmi :
- les dérivés de la kératine, tels que les hydrolysats de kératine et les kératines sulfoniques,
- les dérivés de chitine ou de chitosane anioniques, cationiques, amphotères ou non ioniques,
- les dérivés de cellulose, tels que l'hydroxyéthylcellulose, l'hydroxypropylcellulose, la méthylcellulose, l'éthylhydroxyéthylcellulose, la carboxyméthylcellulose, et les dérivés quaternisés de la cellulose,
- les polymères acryliques, tels que les polyacrylates et les polyméthacrylates, et les copolymères acryliques,
- les copolymères vinyliques non hydroxylés tels que la polyvinylpyrrolidone et ses copolymères, le copolymère de l'éther méthylvinylique et de l'anhydride maléique éventuellement monoestérifié ou monoamidifié ou le copolymère de l'acétate de vinyle et de l'acide crotonique,
- les polymères naturels tels que les gommes arabiques, la gomme de guar et de caroube, les dérivés du xanthane et la gomme de karaya, les alginates et les carraghénates, les glycoaminoglycanes, l'acide hyaluronique et ses dérivés, et
- les polyuréthanes et leurs copolymères.

12. Composition selon l'une quelconque des revendications précédentes, caractérisée par le fait que la phase fluorée comprend en outre au moins un composé fluoré additionnel choisi parmi les perfluoropolyéthers et les composés organofluorés hydrocarbonés résultant d'un hydrocarbure dont moins de 70 % des atomes d'hydrogène sont substitués par des atomes de fluor.

13. Composition selon la revendication 12, caractérisée par le fait que le composé fluoré additionnel est présent en une proportion comprise entre 0,01 et 50 % et de préférence entre 0,1 et 20 % en poids par rapport au poids total de la phase fluorée.

14. Composition selon l'une quelconque des revendications précédentes caractérisée par le fait que la taille des particules de la dispersion est comprise entre 0,2 et 50 µm, et de préférence entre 0,5 et 10 µm.

15. Composition selon l'une quelconque des revendications précédentes, caractérisée par le fait qu'elle renferme en outre de 0,01 à 30 % en poids par rapport au poids total de la composition d'au moins un ingrédient cosmétique ou dermatologique choisi parmi les agents hydratants, les plastifiants, les amincissants, les agents anti-rides, les stimulants, les revitalisants, les raffermissants, les adoucissants, les épaississants, les huiles émulsionnées, les polymères non filmogènes, les conservateurs, les séquestrants, les agents anti-mousse, les parfums, les colorants, les pigments et les charges.

16. Composition selon l'une quelconque des revendications précédentes, caractérisée par le fait qu'elle renferme en outre un alcool de bas poids moléculaire en une proportion comprise entre 0,01 et 20 %.

17. Composition selon l'une quelconque des revendications précédentes, caractérisée par le fait qu'elle se présente sous forme d'un gel, d'un masque, d'une crème, d'un eye-liner, d'un mascara, d'un vernis à ongles aqueux, d'une laque à l'eau, ou d'un produit de coloration temporaire des cheveux.

18. Procédé de préparation d'une composition selon l'une quelconque des revendications 1 à 17, caractérisé par le fait que l'on dissout dans l'eau de 0,05 à 55 % et de préférence de 0,5 à 30 % en poids par rapport au poids total de la composition dudit polymère filmogène hydrosoluble et ensuite après addition éventuelle des additifs cosmétiques ou dermatologiques, on disperse, sous vive agitation, dans la solution obtenue de 0,01 à 70 %, et de préférence 0,1 à 50 % en poids par rapport au poids total de la composition, au moins un composé organofluoré hydrocarboné qui est un hydrocarbure dont au moins 70 % et de préférence au moins 95 % des atomes d'hydrogène ont été substitués par des atomes de fluor.

## Claims

1. Cosmetic or dermatological composition in the form of an oil-in-water dispersion capable of forming a composite film by evaporation of the aqueous phase, characterized in that it comprises a fluorine-containing phase consisting of at least one organic fluorine-containing hydrocarbon compound which is a hydrocarbon at least 70 % and preferably at least 95 % of whose hydrogen atoms have been substituted by fluorine atoms, the said phase being dispersed in an aqueous solution of at least one water-soluble film-forming polymer devoid of units of the following formula:

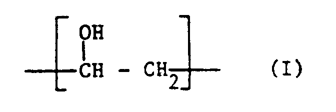

2. Composition according to Claim 1, characterized in that the fluorine-containing phase is present in a concentration of between 0.01 and 70 % and preferably between 0.1 and 50 % by weight relative to the total weight of the composition.

3. Composition according to Claim 1 or 2, characterized in that the fluorine-containing phase comprises at least one organic fluorine-containing hydrocarbon compound, which is solid at room temperature, dissolved in at least one organic fluorine-containing hydrocarbon compound which is liquid at room temperature.

4. Composition according to any one of the preceding claims, characterized in that the organic fluorine-containing hydrocarbon compound is a perfluorinated organic hydrocarbon compound chosen from perfluoroalkanes, perfluorocycloalkanes, perfluoro(alkylcycloalkanes), perfluoropolycycloalkanes, perfluoroarenes and perfluorinated organic hydrocarbon compounds comprising at least one heteroatom.

5. Composition according to Claim 4, characterized in that the perfluorinated organic hydrocarbon compound is chosen from perfluorooctane, perfluorononane, perfluorodecane, perfluorodecalin, perfluoro(methyldecalin), perfluoro(butylcyclohexane), perfluorotrimethylbicyclo[3.3.1]nonane, perfluorodimethyladamantane, tetracosafluorotetradecahydrophenanthrene, perfluoronaphthalene and perfluoromethyl-1-naphthalene.

6. Composition according to Claim 4, characterized in that the perfluorinated organic hydrocarbon compound comprising at least one heteroatom is chosen from perfluoro(tertiary amines), perfluoro(alkyltetrahydrofurans), perfluoro(alkyltetrahydropyrans) and the perfluorinated derivatives of morpholine.

7. Composition according to Claim 6, characterized in that the perfluorinated organic hydrocarbon compound comprising at least one heteroatom is chosen from perfluorotributylamine, perfluoro(butyltetrahydrofuran), perfluoro(hexyltetrahydropyran) and perfluoro(N-pentylmorpholine).

8. Composition according to any one of Claims 1 to 3, characterized in that the organic fluorine-containing hydrocarbon compound is a partially fluorinated organic hydrocarbon compound of formula:
(RF)ₓ-(A)_{y}-(RH)_{z} (II)
in which:
x can be 1, 2 or 3;
y can be 0 or 1;
z can be 0, 1, 2 or 3;
y and z cannot simultaneously be zero,
and, where z = 0, then y = 1 and x = 2 or 3;
RF represents an aromatic or aliphatic, saturated or unsaturated fluorine-containing radical whose chain can be linear, branched or cyclic, it being possible for the chain to be optionally functionalized and/or to be interrupted by divalent atoms such as oxygen or sulphur, or by trivalent atoms such as nitrogen, it being possible in addition for the chain to be substituted by hydrogen atoms or other halogen atoms, with the proviso that no more than one of these substituents, other than fluorine, is present for each two carbon atoms,
RH represents an aliphatic or aromatic, saturated or unsaturated hydrocarbon radical whose chain can be linear, branched or cyclic and can optionally be functionalized and/or interrupted by divalent atoms such as oxygen or sulphur or by trivalent atoms such as nitrogen, and
A represents a covalent bond when y = 0 and, when y is other than 0, A represents a divalent or polyvalent radical.

9. Composition according to Claim 8, characterized in that the partially fluorinated hydrocarbon compound corresponds to one of the following formulae:
a) 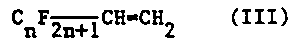 in which: n is 6 or 8
b) 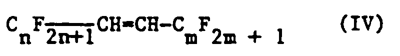 in which: n and m independently are integers between 2 and 10 and the sum n + m is greater than or equal to 8
c)
C₈F₁₇-C₂H₅ (V),
d) 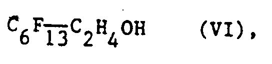 and
e)

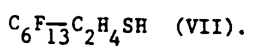

10. Composition according to any one of the preceding claims, characterized in that the water-soluble film-forming polymer is present in a proportion of between 0.05 and 55 % and preferably between 0.5 and 30 % by weight relative to the total weight of the composition.

11. Composition according to any one of the preceding claims, characterized in that the water-soluble film-forming polymer is chosen from:
- keratin derivatives, such as keratin hydrolysates and sulphonic keratins,
- anionic, cationic, amphoteric or nonionic chitin or chitosan derivatives,
- cellulose derivatives, such as hydroxyethylcellulose, hydroxypropylcellulose, methylcellulose, ethylhydroxyethylcellulose, carboxymethylcellulose and quaternized derivatives of cellulose,
- acrylic polymers, such as polyacrylates and polymethacrylates, and acrylic copolymers,
- non-hydroxylated vinyl copolymers, such as polyvinylpyrrolidone and its copolymers, the optionally monoesterified or monoamidated copolymer of methyl vinyl ether and maleic anhydride, or the copolymer of vinyl acetate and crotonic acid,
- natural polymers such as gum arabics, guar gum and carob gum, xanthan derivatives and karaya gum, alginates and carrageenans, glycoaminoglycans, hyaluronic acid and its derivatives, and
- polyurethanes and their copolymers.

12. Composition according to any one of the preceding claims, characterized in that the fluorine-containing phase additionally comprises at least one additional fluorine-containing compound chosen from perfluoropolyethers and the organic fluorine-containing hydrocarbon compounds resulting from a hydrocarbon at least 70 % of whose hydrogen atoms are substituted by fluorine atoms.

13. Composition according to Claim 12, characterized in that the additional fluorine-containing compound is present in a proportion of between 0.01 and 50 % and preferably between 0.1 and 20 % by weight relative to the total weight of the fluorine-containing phase.

14. Composition according to any one of the preceding claims, characterized in that the particle size of the dispersion is between 0.2 and 50 µm, and preferably between 0.5 and 10 µm.

15. Composition according to any one of the preceding claims, characterized in that it additionally contains from 0.01 to 30 % by weight, relative to the total weight of the composition, of at least one cosmetic or dermatological ingredient chosen from moisturizers, plasticizers, slimming agents, anti-wrinkle agents, stimulants, revitalization agents, toning agents, emollients, thickeners, emulsified oils, non-film-forming polymers, preservatives, sequestrants, antifoams, fragrances, dyes, pigments and fillers.

16. Composition according to any one of the preceding claims, characterized in that it additionally comprises an alcohol of low molecular weight in a proportion of between 0.01 and 20 %.

17. Composition according to any one of the preceding claims, characterized in that it is provided in the form of a gel, face pack, cream, eyeliner, mascara, aqueous nail varnish, water-containing lacquer or of a product for temporary dyeing of the hair.

18. Process for the preparation of a composition according to any one of Claims 1 to 17, characterized in that from 0.05 to 55 % and preferably 0.5 to 30 % by weight, relative to the total weight of the composition, of the said water-soluble film-forming polymer are dissolved in water and then, after optional addition of the cosmetic or dermatological additives, from 0.01 to 70 % and preferably from 0.1 to 50 % by weight, relative to the total weight of the composition, of at least one organic fluorine-containing hydrocarbon compound which is a hydrocarbon at least 70 % and preferably at least 95 % of whose hydrogen atoms have been substituted by fluorine atoms, is dispersed with vigorous stirring in the solution obtained.

## Patentansprüche

1. Kosmetische oder dermatologische Zubereitung in Form einer Öl-in-Wasser-Dispersion, die dazu befähigt ist, durch Evaporation der wäßrigen Phase einen zusammenhängenden Film zu bilden, dadurch gekennzeichnet, daß sie eine mindestens aus einem organischen Fluorkohlenwasserstoff bestehende fluorierte Phase enthält, wobei in dem Kohlenwasserstoff mindestens 70 %, vorzugsweise mindestens 95 % der Wasserstoffatome durch Fluoratome ersetzt sind, und diese Phase in einer wäßrigen Lösung aus mindestens einem filmbildenden wasserlöslichen Polymer, das frei von Einheiten der folgenden Formel (I) ist, dispergiert ist;

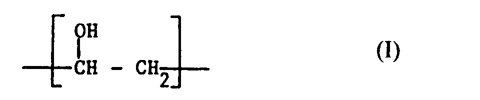

2. Zubereitung gemäß Anspruch 1, dadurch gekennzeichnet, daß die fluorierte Phase in einer Konzentration von 0,01 bis 70 Gew.-%, vorzugsweise von 0,1 bis 50 Gew.-%, bezogen auf das Gesamtgewicht der Zubereitung, vorliegt.

3. Zubereitung gemäß Anspruch 1 oder 2, dadurch gekennzeichnet, daß die fluorierte Phase mindestens einen bei Raumtemperatur festen organischen Fluorkohlenwasserstoff solubilisiert in mindestens einem bei Raumtemperatur flüssigen organischen Fluorkohlenwasserstoff enthält.

4. Zubereitung gemäß einem der vorstehenden Ansprüche, dadurch gekennzeichnet, daß es sich bei dem organischen Fluorkohlenwasserstoff um eine perfluorierte organische Kohlenwasserstoffverbindung handelt, die aus Perfluoralkanen, Perfluorcycloalkanen, Perfluoralkylcycloalkanen, Perfluorpolycycloalkanen, Perfluorarenen und mindestens ein Heteroatom tragenden, organischen perfluorierten Kohlenwasserstoffen ausgewählt ist.

5. Zubereitung gemäß Anspruch 4, dadurch gekennzeichnet, daß die perfluorierte organische Kohlenwasserstoffverbindung aus Perfluoroctan, Perfluornonan, Perfluordecan, Perfluordecalin, Perfluor(methyldecalin), Perfluor(butylcyclohexan), Perfluor(trimethylbicyclo[3.3.1]nonan), Perfluor(dimethyladamantan), Tetracosafluortetradecahydrophenantren, Perfluornaphthalin und Perfluormethyl-1-naphthalin ausgewählt ist.

6. Zubereitung gemäß Anspruch 4, dadurch gekennzeichnet, daß die mindestens ein Heteroatom tragende, perfluorierte organische Kohlenwasserstoffverbindung aus den Perfluor-(t-aminen), Perfluor(alkyltetrahydrofuranen), Perfluor(alkyltetrahydropyranen) und perfluorierten Morpholinderivaten ausgewählt ist.

7. Zubereitung gemäß Anspruch 6, dadurch gekennzeichnet, daß die mindestens ein Heteroatom tragende, perfluorierte organische Kohlenwasserstoffverbindung aus Perfluortributylamin, Perfluor(butyltetrahydrofuran), Perfluor(hexyltetrahydroppyran) und Perfluor(N-pentylmorpholin) ausgewählt ist.

8. Zubereitung gemäß einem der Ansprüche 1 bis 3, dadurch gekennzeichnet, daß es sich bei der organischen Fluorkohlenwasserstoffverbindung um einen teilweise fluorierten Kohlenwasserstoff der Formel (II) handelt:
(RF)ₓ-(A)_{y}-(RH)_{z} (II)
in der
x 1,2 oder 3
y 0 oder 1 und
z 0, 1, 2 oder 3 sein kann,
wobei y und z nicht gleichzeitig 0 sein können und dann, wenn z = 0 ist, y = 1
und X = 2 oder 3 sind, und
RF einen fluorierten, gesättigten oder ungesättigten aromatischen oder aliphatischen Rest darstellt, wobei die Kette linear, verzweigt oder cyclisch sein, eventuell eine funktionelle Gruppe tragen und/oder durch zweiwertige Atome wie Sauerstoff oder Schwefel oder dreiwertige Atome wie Stickstoff unterbrochen sein kann, die Kette weiterhin mit Wasserstoffatomen oder anderen Halogenen substituiert sein kann, zwar unter der Bedingung, daß nicht mehr als ein Substituent verschieden von Fluor pro 2 Kohlenstoffatome vorhanden ist,
RH einen fluorierten, gesättigten oder ungesättigten aromatischen oder aliphatischen Rest darstellt, wobei die Kette linear, verzweigt oder cyclisch sein, eventuell eine funktionelle Gruppe tragen und/oder durch zweiwertige Atome wie Sauerstoff oder Schwefel oder dreiwertige Atome wie Stickstoff unterbrochen sein kann, und A dann, wenn y = 0, eine kovalente Bindung und dann, wenn y nicht 0 ist, einen zwei- oder mehrwertigen Rest darstellt.

9. Zubereitung gemäß Anspruch 8, dadurch gekennzeichnet, daß die teilweise fluorierte Kohlenwasserstoffverbindung einer der folgenden Formlen entspricht:
a) 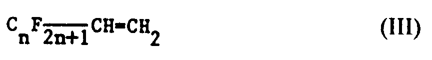 in der n 6 oder 8 ist,
b) 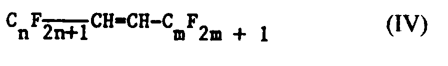 in der n und m unabhängig voneinander ganze Zahlen zwischen 2 und 10 sind und die Summe n + m größer oder gleich 8 ist,
c)
C₈F₁₇-C₂H₅ (V)
d) 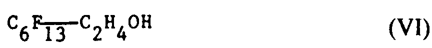 und
e)

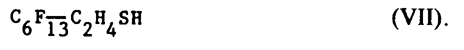

10. Zubereitung gemäß einem der vorstehenden Ansprüche, dadurch gekennzeichnet, daß das wasserlösliche filmbildende Polymer in einer Menge zwischen 0,05 und 55 Gew.-% und vorzugsweise zwischen 0,5 und 30 Gew.-% vorhanden ist, bezogen auf das Gesamtgewicht der Zubereitung.

11. Zubereitung gemäß einem der vorstehenden Ansprüche, dadurch gekennzeichnet, daß das wasserlösliche filmbildende Polymer ausgewählt ist aus:
- Keratinderivaten wie Keratinhydrolysaten und schwefelhaltigem Keratin,
- anionischen, kationischen, amphoteren oder nichtionischen Chitin-oder Chitosanderivaten,
- Cellulosederivaten wie Hydroxyethylcellulose, Hydroxypropylcellulose, Methylcellulose, Ethylhydroxyethylcellulose, Carboxymethylcellulose sowie quarternären Cellulosederivaten,
- Acrylpolymeren wie Polyacrylaten und Polymethacrylaten sowie Acryl-Copolymeren,
- keine Hydroxylgruppen enthaltenden Vinyl-Copolymeren wie Polyvinylpyrrolidon und dessen Copolymeren, Methylvinylether-Copolymeren sowie Maleinsäureanhydrid, das wahlweise in Form des Monoesters oder Monoamids vorliegen kann, oder Vinylacetat/Crotonsäure-Copolymer,
- natürlichen Polymeren wie Gummi arabicum, Guar- oder Johannisbrot-Gummi, Xanthanderivaten und Karaya-Gummi, Alginaten und Carrageenaten, Glucosaminoglykanen, Hyaluronsäure und deren Derivaten und
- den Polyurethanen und deren Copolymeren.

12. Zubereitung gemäß einem der vorstehenden Ansprüche, dadurch gekennzeichnet, daß die fluorierte Phase zusätzlich eine weitere fluorierte Verbindung enthält, die aus Perfluorpolyethern und solchen organischen Fluorkohlenwasserstoffverbindungen ausgewählt ist, die aus einem Kohlenwasserstoff hervorgegangen sind, bei dem mindestens 70 % der Wasserstoffatome durch Fluoratome ersetzt sind.

13. Zubereitung gemäß Anspruch 12, dadurch gekennzeichnet, daß die weitere fluorierte Verbindung in einer Menge von 0,01 bis 50 Gew.-%, vorzugsweise von 0,1 bis 20 Gew.-%, bezogen auf das Gesamtgewicht der fluorierten Phase, vorhanden ist.

14. Zubereitung gemäß einem der vorstehenden Ansprüche, dadurch gekennzeichnet, daß der Durchmesser der Teilchen der Dispersion zwischen 0,2 und 50 µm, vorzugsweise zwischen 0,5 und 10 µm liegt.

15. Zubereitung gemäß einem der vorstehenden Ansprüche, dadurch gekennzeichnet, daß sie zusätzlich 0,01 bis 30 Gew.-%, bezogen auf das Gesamtgewicht der Zubereitung, mindestens eines kosmetischen oder dermatologischen Bestandteils enthält, der aus Feuchtigkeitsspendern, Weichmachern, Schlankheitsmitteln, Wirkstoffen gegen Falten, Stimulanzien, revitalisierenden, straffenden und glättenden Mitteln, Verdickungsmitteln, emulgierenden Ölen, filmbildenden Polymeren, Konservierungsmitteln, Sequestriermitteln, Schaumbremsmitteln, Parfümen, Farbstoffen, Pigmenten und Trägerstoffen ausgewählt ist.

16. Zubereitung gemäß einem der vorstehenden Ansprüche, dadurch gekennzeichnet, daß sie außerdem einem Alkohol niedrigen Molekulargewichts in einer Menge von 0,01 bis 20 Gew.-% enthält.

17. Zubereitung gemäß einem der vorstehenden Ansprüche, dadurch gekennzeichnet, daß sie in Form eines Gels, einer Maske, einer Creme, eines Eye-Liners, einer Wimperntusche, eines wäßrigen Nagellacks, eines Haarsprays auf Wasserbasis oder eines Produkts zum vorübergehenden Färben der Haare vorliegt.

18. Verfahren zur Herstellung einer Zubereitung gemäß einem der vorstehenden Ansprüche 1 bis 17, dadurch gekennzeichnet, daß 0,05 bis 55 Gew.-%, vorzugsweise 0,5 bis 30 Gew.-%, bezogen auf das Gesamtgewicht der Zubereitung, an wasserlöslichem filmbildenden Polymeren in Wasser gelöst und in der erhaltenen Lösung nach der Zugabe eventueller kosmetischer oder dermatologischer Additive unter heftigem Rühren 0,01 bis 70 Gew.-%, vorzugsweise 0,1 bis 50 Gew.-%, bezogen auf das Gesamtgewicht der Zubereitung, mindestens an einer organischen Fluorkohlenwasserstoffverbindung dispergiert werden, wobei es sich um einem Kohlenwasserstoff handelt, bei dem mindestens 70 %, vorzugsweise mindestens 95 % der Wasserstoffatome durch Fluoratome substituiert sind.
